# EUROPEAN PATENT SPECIFICATION

(11) **EP 0 574 088 B1**
(45) Date of publication and mention of the grant of the patent: **11.09.1996**
(21) Application number: 93201639.7
(22) Date of filing: 08.06.1993
(51) Int. Cl.: C07C 323/52, C10M 135/26

(54) **Salts of mercaptocarboxylic acid derivatives and their use as additives for lubricating oils**
Salze von Mercaptocarbonsäure-Derivaten sowie deren Verwendung als Additive für Schmieröle
Sels des dérivés des acides mercaptocarboxyliques et leur utilisation comme additifs pour huiles lubrifiantes

(30) Priority: 09.06.1992 EP 92305281
(43) Date of publication of application: 15.12.1993
(73) Proprietor: SHELL INTERNATIONALE RESEARCH MAATSCHAPPIJ B.V., NL-2596 HR Den Haag (NL)
(72) Inventor: Udding, Anne Catharinus, Faversham, Kent ME13 8LP (GB); Van Der Hulst, Cornelis Hyacinthus Maria, Coxheath, Kent ME17 4DQ (GB)

(56) References cited:
- EP-A- 0 248 465
- US-A- 3 741 909
- PATENT ABSTRACTS OF JAPAN vol. 6, no. 68 (C-100)30 April 1982 & JP-A-57 005 740 (SANKYO YUKI GOSEI) 12 January 1982
- PATENT ABSTRACTS OF JAPAN vol. 6, no. 15 (C-89)28 January 1982 & JP-A-56 139 557 (SANKYO YUKI GOSEI) 31 October 1981
- PATENT ABSTRACTS OF JAPAN vol. 6, no. 15 (C-89)28 January 1982 & JP-A-56 139 558 (SANKYO YUKI GOSEI) 31 October 1981
- CHEMICAL ABSTRACTS, vol. 81, no. 20, 18 November 1974, Columbus, Ohio, US; abstract no. 122395a, page 100 ; & JP-A-49 001 934 (LION FAT AND OIL) 17 January 1974
- CHEMICAL ABSTRACTS, vol. 77, no. 18, 30 October 1972, Columbus, Ohio, US; abstract no. 115477t, page 45 ; & JP-A-47 024 541 (MITSUBISHI RAYON) 6 July 1972
- CHEMICAL ABSTRACTS, vol. 77, no. 25, 18 December 1972, Columbus, Ohio, US; abstract no. 164043g, page 360 ; & JP-A-47 024 005 (MITSUBISHI RAYON) 3 July 1972
- PATENT ABSTRACTS OF JAPAN vol. 6, no. 68(C-100)30 April 1982 & JP-A-57 005 741 (SANKYO YUKI GOSEI) 12 January 1982

## Description

The present invention relates to salts, in particular calcium and magnesium salts, of certain mercaptocarboxylic acid derivatives, a process for their preparation, and lubricating oil compositions and concentrates containing them.

It is known from GB-A-532676 to prepare thioethers and/or mercaptans by reacting unsaturated hydrocarbons with compounds of the general formula RSH where R is hydrogen or an organic radical in the presence of peroxides and of iron, chromium, magnesium, aluminium, thorium, uranium, cerium, lanthanum, beryllium, osmium, molybdenum, vanadium and manganese salts of strong, inorganic acids. As examples of unsaturated hydrocarbons are mentioned ethylene, butene-1, butene-2, hexene-1, cetene, acetylene, hexyne-1, cetyn-2, styrene, phenyl acetylene, cyclohexene, butadiene and pinene. Thioglycolic acid is mentioned as an example of a compound of the general formula RSH. GB-A-532676 contains no disclosure of any use of the resulting thioethers or mercaptans.

GB-A-835987 discloses lubricating compositions comprising a major proportion of a lubricating oil and a synergistic admixture of from 0.005 to 1.0%w each of a branched C₉ to C₁₇ alkylmercaptoacetic acid and a barium or calcium C₆ to C₁₂ branched chain dialkyl naphthalene sulphonate, to impart superior rust inhibition to the composition. There is no suggestion that any derivative of the alkylmercaptoacetic acid might be used in place of or in addition to the acid per se.

US-A-3741909 discloses compositions prepared by mixing an organic material with a compound of the general formula (R-S-X-COO)ₙM where R is a C₆-C₂₂ hydrocarbon radical, X is a C₁-C₅ alkylene radical, n is 1 to 5, and M is a non-alkali metal, along with a phenol-type or amine-type antioxidant. However, the only compounds of that general formula exemplified are (C₁₆H₃₃SCH₂CH₂COO)₂Ca and (C₁₆H₃₃SCH₂COO)₂Mg.

In accordance with the present invention there are provided alkaline earth metal salts selected from calcium and magnesium salts of a mercaptocarboxylic acid derivative of the general formula

R-S-(CR¹R²)ₙ-COOH (I)

in which n is 1 to 10; R represents an alkyl group, optionally substituted by a cycloalkyl, heterocyclyl or aryl group; or a cycloalkyl group optionally substituted by an alkyl group ; and R¹ and R² are independently selected from hydrogen atoms, and hydroxyl, carboxyl, alkyl, alkoxy, alkylthio, cycloalkyl, aralkyl and aryl groups; subject to the total number of carbon atoms in the derivative being in the range from 12 to 50; with the proviso that the salt excludes calcium salts in which (a) n is 1, R¹ and R² are each a hydrogen atom and R is a C₈, C₁₂ or C₂₀ alkyl group or a benzyl group; (b) n is 2, each R¹ and R² is a hydrogen atom and R is a C₁₂ or C₁₆ alkyl group; and (c) n is 2, each R¹ is a hydrogen atom, the R² on the carbon atom adjacent to the sulphur atom is a hydrogen atom and the other R² is a methyl group, and R is a C₁₂ alkyl group, and excludes magnesium salts in which (a) n is 1, R¹ and R² are each a hydrogen atom and R is a C₁₆ alkyl group; (b) n is 2, each R¹ and R² is a hydrogen atom and R is a C₈, C₁₂ or C₁₈ alkyl group or a benzyl group; and (c) n is 2, each R¹ is a hydrogen atom, the R² on the carbon atom adjacent to the sulphur atom is a hydrogen atom and the other R² is a methyl group, and R is a C₁₂ or C₁₈ alkyl group.

The mercaptocarboxylic acid derivative of formula I preferably contains from 12 to 30, and especially 16 to 21, carbon atoms.

In this specification, unless otherwise stated, an alkyl group or alkyl moiety in an alkoxy, alkylthio or aralkyl group may be linear or branched and preferably contains up to 6, more preferably up to 10, and especially up to 20, carbon atoms. The substituent(s) in a substituted, branched alkyl group may be located either on the main chain or on the side-chain(s) of the alkyl group. A cycloalkyl group may contain from 3 to 8, preferably 3 to 6, carbon atoms. A heterocyclyl group may be any saturated or unsaturated ring system, e.g. a C₅₋₇ ring system, containing at least one heteroatom selected from oxygen; nitrogen and sulphur, 5- and 6-membered rings being especially preferred, e.g. a tetrahydrofuranyl, furanyl, piperidinyl, pyridinyl, tetrahydrothiophenyl or thiophenyl (thienyl) group. An aryl group may be any aromatic hydrocarbon group, especially a phenyl or naphthyl group.

The preferred salts of the present invention are calcium salts.

In formula I above, n preferably ranges from 1 to 6, more preferably from 1 to 4, and is especially 1.

R preferably represents a C₁-C₂₀ alkyl group, optionally substituted by one or more substituents selected from C₃-C₈ cycloalkyl, tetrahydrofuranyl, furanyl, piperidinyl, pyridinyl, tetrahydrothiophenyl, thiophenyl, phenyl and naphthyl groups; or a C₃-C₈ cycloalkyl group optionally substituted by one or more C₁-C₁₀ alkyl groups.

More preferably, R represents a C₁₀-C₂₀ alkyl group, optionally substituted by one or two substituents selected from C₃-C₆ cycloalkyl, tetrahydrofuranyl, furanyl, piperidinyl, pyridinyl, tetrahydrothiophenyl, thiophenyl, phenyl and naphthyl groups; or a C₃-C₆ cycloalkyl group optionally substituted by one or two C₁-C₆ alkyl groups.

Particularly preferred derivatives of formula I above are those in which R represents a C₁₄-C₁₉ alkyl group.

Preferably R¹ and R² are independently selected from hydrogen atoms, and hydroxyl, carboxyl, C₁-C₁₀ alkyl, C₁-C₁₀ alkoxy, C₁-C₁₀ alkylthio, C₃-C₈ cycloalkyl, benzyl, phenyl and naphthyl groups.

More preferably R¹ and R² are independently selected from hydrogen atoms, and hydroxyl, carboxyl, C₁-C₆ alkyl, C₁-C₆ alkoxy, C₁-C₆ alkylthio, C₃-C₆ cycloalkyl, benzyl, phenyl and naphthyl groups.

Most preferably, R¹ and R² represent hydrogen atoms.

It will be appreciated that when n is greater than 1, the -CR¹R² groups can be the same as or different from one another.

A particularly preferred sub-group of compounds of formula I is that in which n is 1, R represents a C₁₄-C₁₉ alkyl group and each of R¹ and R² represents a hydrogen atom.

The calcium and magnesium salts of the present invention may be neutral salts, i.e. those containing stoichiometrically equivalent amounts of metal and carboxylate moieties, or they may be overbased salts, as hereinafter defined. Overbased salts are preferred.

The present invention further provides a process for the preparation of an alkaline earth metal salt selected from calcium and magnesium salts of a mercaptocarboxylic acid derivative of formula I as defined above, which comprises reacting a mercaptocarboxylic acid derivative of formula I above with an oxide or hydroxide of calcium or magnesium.

The mercaptocarboxylic acid derivative of formula I is conveniently dissolved in an organic solvent and the resulting solution mixed with a stoichiometric equivalent amount of a suspension of a hydroxide and/or oxide of calcium or magnesium conveniently in the same solvent to yield the neutral calcium or magnesium salt of the formula I compound. The presence of an initial, small amount of water is beneficial in that it enhances the rate of reaction. The reaction rate is also enhanced by removing water formed during the reaction azeotropically. The neutral salt is subsequently purified according to conventional known techniques and, if desired, converted to an overbased salt by mixing in an organic solvent with further of the hydroxide and/or oxide of calcium or magnesium, a promoter and a small amount of water and passing carbon dioxide through the reaction mixture.

The overbased salt can, alternatively, be obtained directly from the mercaptocarboxylic acid derivative of formula I by treating the derivative in the same way as the neutral salt as described above.

In the present context, an overbased salt denotes a salt in which the basicity index (BI), defined as the equivalent ratio of total calcium or magnesium to calcium or magnesium salt of the mercaptocarboxylic acid derivative of formula I (as determined by potentiometric titration), is greater than 1. Detailed descriptions of overbasing processes are given in many patent specifications, for example GB-A-786,167.

The organic solvent may conveniently be a hydrocarbon, such as an aromatic hydrocarbon or a hydrocarbon fraction rich in aromatics, such as gasoline, with compounds such as benzene, toluene, or especially, xylene being preferred.

The promoter may, for example, be a C₁-C₃ alcohol, preferably methanol. The amount of water used is conveniently in the range from 0.5 to 1.5 mol per equivalent mercaptocarboxylic acid derivative, or calcium or magnesium salt thereof.

Certain of the mercaptocarboxylic acid derivatives of formula I are known materials, e.g. from GB-A-532,676 discussed above.

The mercaptocarboxylic acid derivatives of formula I as defined above may conveniently be prepared, for example, as described in GB-A-532,676, by reacting, in the presence of a peroxide, a compound of the general formula:

R'-H (II)

with a compound of the general formula:

HS-(CR¹R²)ₙ-COOH (III)

wherein n is 1 to 10; R' represents an alkenyl group, optionally substituted by a cycloalkyl, heterocyclyl or aryl group; or a cycloalkenyl group optionally substituted by an alkyl group; and R¹ and R² are as defined above; subject to the total number of carbon atoms in the compounds of formulae II and III taken together being in the range from 12 to 50.

The compounds of formula I above may also conveniently be prepared from the compounds of formulae II and III above by the methods of Rühlmann, Gramer, Heuchel and Schräpler, J. Prakt. Chem., (4) 10, 316 (1960); Weibull, Arkiv Kemi. Mineral. Geol., 23A, No. 18 (1946); Henkel and Cie GmbH, GB-A-470,717 [Chem. Abs., 32, 593 (1938)]; Smith and Hernestam, Acta Chem. Scand., 8, 1111 (1954); Holmberg, Arkiv Kemi. Mineral. Geol., 15A, No. 21 (1942); Cunneen, J. Chem. Soc., 1947, 36 (references 466, 468, 481, 483, 490 and 108 respectively which are listed in Table XB, pp. 262-267 in "Organic Reactions", Volume 13 (John Wiley & Sons Inc., New York, London, 1963)); or by processes analogous thereto.

Certain mercaptocarboxylic acid derivatives of formula I as defined above, wherein n is at least 2, may alternatively be prepared by reacting, in the presence of ultra-violet light and, optionally, oxygen and a mercury salt, a compound of the general formula:

R-SH (IV)

with a compound of the general formula:

CR¹R² = CR¹(CR¹R²)₍ₙ₋₂₎-COOH (V)

wherein n, R, R¹ and R² are as defined above, subject to the total number of carbon atoms in the compounds of formulae IV and V taken together being in the range from 12 to 50, e.g. according to the methods of Kaneko and Mii, J. Chem. Soc. Japan, 59, 1382 (1938) [Chem. Abs., 33, 2106 (1939)]; Koenig and Swern, J. Am. Chem. Soc., 79, 4235 (1957) (references 183 and 456 listed in Table XA, pp. 247-261 in "Organic Reactions", Volume 13, (John Wiley & Sons Inc., New York, London, 1963)); or by processes analogous thereto.

The compounds of formulae II, III, IV and V are known compounds or can be prepared by processes analogous to known processes.

The alkaline earth metal salts according to the present invention may be used as detergent additives in lubricating oils. Accordingly, the present invention further provides a lubricating oil composition comprising a major proportion of a lubricating oil and a minor proportion, preferably from 0.1 to 20%w, more preferably from 0.1 to 10%w, and especially from 0.2 to 5%w, based on the total composition, of an alkaline earth metal salt selected from calcium and magnesium salts of a mercaptocarboxylic acid derivative of formula I as defined above.

Suitable lubricating oils are natural, mineral or synthetic lubricating oils.

Natural lubricating oils include animal and vegetable oils, such as castor oil. Mineral oils comprise the lubricating oil fractions derived from crude oils, coal or shale, which fractions may have been subjected to certain treatments such as clay-acid, solvent or hydrogenation treatments. Synthetic lubricating oils include synthetic polymers of hydrocarbons, modified alkylene oxide polymers, and ester lubricants, which are known in the art. These lubricating oils are preferably crankcase lubricating oils for spark-ignition and compression-ignition engines, but include also hydraulic lubricants, metal-working fluids and automatic transmission fluids.

Preferably the lubricating base oil component of the compositions according to the present invention is a mineral lubricating oil or a mixture of mineral lubricating oils, such as those sold by member companies of the Royal Dutch/Shell Group under the designations "HVI", or "XHVI" (Trade Mark).

The viscosity of the lubricating base oils present in the compositions according to the present invention may vary within wide ranges, and is generally from 3 to 35 mm²/s at 100°C.

The lubricating oil compositions of the present invention may further contain a number of other additives, such as antioxidants, anti-wear agents, ashless dispersants such as polyolefin-substituted succinimides, e.g. those described in GB-A-2 231 873, friction modifiers, foam inhibitors, corrosion inhibitors, viscosity index improvers, and pour point depressants, as can be established by a person skilled in the art.

The present invention still further provides a lubricating oil concentrate comprising from 10 to 80%w, based on the total concentrate, of an alkaline earth metal salt selected from calcium and magnesium salts of a mercaptocarboxylic acid derivative of formula I as defined above. Such a concentrate generally comprises an inert carrier fluid and one or more additives in a concentrated form. The inert carrier fluid is conveniently a lubricating oil.

The present invention still further provides a process for preparing a lubricating oil composition which comprises mixing a lubricating base oil with an alkaline earth metal salt selected from calcium and magnesium salts of a mercaptocarboxylic acid derivative of formula I as defined above, or with a lubricating oil concentrate according to the invention.

The invention will be further understood from the following illustrative examples.

### Example 1

### (i) Preparation of Linear C₁₄-C₁₈-alkylmercaptoacetic acids

Into a 11 three-necked round bottomed flask provided with a stirrer, thermowell and condensor were introduced 160g (1.74 mol) mercaptoacetic acid, 311g (1.39 mol) of a mixture of C₁₄-C₁₈- alpha olefins derived from the "SHELL" Higher Olefins Process (the molar ratio C₁₄:C₁₆:C₁₈ being 1:2:1) and 0.33g lauryl peroxide. The reaction mixture was heated to 80°C with vigorous stirring and the temperature rose to 110°C as a result of exothermic reaction. Further portions of 0.33g lauryl peroxide were added to the reaction mixture after 1½ and 5 hours reaction at 80°C. The total reaction time at 80°C was 7½ hours. The reaction mixture was subsequently distilled at 100 to 200°C at a pressure of 200Pa to remove unreacted olefins and mercaptoacetic acid. 396g (89% yield based on olefin) of a mixture of linear C₁₄-C₁₈-alkylmercaptoacetic acids were obtained as a solid residue melting around 80°C and having an acid content, as determined by titration with 0.1N potassium hydroxide in isopropanol, of 3.10 mmol/g (theoretical acid content, 3.16 mmol/g).

### (ii) Preparation of Overbased Calcium Salts of Linear C₁₄-C₁₈-alkylmercaptoacetic acids

Into a 31 glass reactor was introduced a solution of 389g (1.21 equivalents) of the mixture of linear C₁₄-C₁₈-alkylmercaptoacetic acids obtained in (i) above in 2023g xylene (0.5 meq. acid/g). To this solution were added 201g (2.71 mol; 5.42 equivalents) calcium hydroxide and the resulting suspension stirred for 1 hour at 40°C. Subsequently, 268g methanol (97%) (10%w, based on total weight of acid, xylene and methanol) were added to the suspension, the reaction temperature was increased to 60°C and 2.05 equivalents carbon dioxide gas were introduced at a rate of 0.08 equivalent carbon dioxide gas per equivalent acid per minute. The equivalent ratio of acid: calcium hydroxide: carbon dioxide gas was 1:4.5:1.7. The slurry thus formed was centrifuged for 2 hours at 2800rpm and at 20°C to yield a precipitate of unreacted calcium hydroxide and two liquid layers, a thin, upper layer containing mainly methanol, and a main, lower layer containing xylene and overbased calcium salts of the linear C₁₄-C₁₈-alkylmercaptoacetic acids. The upper methanol layer was removed, followed by decantation of the lower xylene layer (2091g). Analysis of the xylene layer by acidification with hydrochloric acid in isopropanol and then titration with 0.5N potassium hydroxide in isopropanol showed it to contain 2.92%w calcium and the basicity index of the overbased calcium salts to be 3.63. 621g "HVI 60" base oil (a bright and clear high viscosity index base oil having viscosity at 100°C of 4.4 to 4.9 mm²/s (ASTM D445) and minimum flash point 200°C (ASTM D92)) were then added to the xylene layer and the xylene removed from the oil mixture by distillation at 130°C and 200 Pa pressure to afford an oil concentrate containing the overbased calcium salts. The oil concentrate was found to have a calcium content of 5.92%w and a viscosity at 100°C of 10.1 mm²/s. The basicity index of the overbased calcium salts was 3.59.

### Example 2

### (i) Preparation of Branched C₁₄-C₁₉-alkylmercaptoacetic acids

Into a 21 three-necked round bottomed flask provided with a stirrer, thermowell and condensor were introduced 736g (8 mol) mercaptoacetic acid, 920g (4 mol) of a mixture of C₁₄-C₁₉ internal olefins having average molecular weight 230, derived from the "SHELL" Higher Olefins Process, and 9.2g ditertiarybutyl peroxide. The reaction mixture was heated to 140°C with vigorous stirring. Further portions of 9.2g ditertiarybutyl peroxide were added to the reaction mixture after 2½, 5 and 7½ hours reaction at 140°C. The total reaction time at 140°C was 22 hours. The reaction mixture, which was composed of two liquid layers - an upper and a lower layer, was transferred to a separatory funnel and the lower layer removed and discarded. The remaining upper layer was distilled at 100 to 200°C under vacuum to afford 1055g of a mixture of branched C₁₄-C₁₉-alkylmercaptoacetic acids as a solid residue having an acid content (determined as in Example 1(i) above) of 2.97 meq./g (theoretical acid content 3.11 meq./g).

### (ii) Preparation of Overbased Calcium Salts of Branched C₁₄-C₁₉ -alkylmercaptoacetic acids

Into a 51 glass reactor was introduced a solution of 500g (1485 milli-equivalents) of the mixture of branched C₁₄-C₁₉-alkylmercaptoacetic acids obtained in (i) above in 2470g xylene (0.5 meq. acid/g). To this solution were added 231g (6237 milli-equivalents) calcium hydroxide and the resulting suspension stirred for 1 hour at 50°C. Subsequently, 330g methanol (97%) (10%w, based on total weight of acid, xylene and methanol) were added to the suspension, the reaction temperature was increased to 60°C and 2524 milli-equivalents carbon dioxide gas were introduced at a rate of 0.085 equivalent carbon dioxide gas per equivalent acid per minute. The milli-equivalent ratio of acid: calcium hydroxide: carbon dioxide gas was 1:4.2:1.7. The slurry thus formed was centrifuged for 2 hours at 2400 rpm and at 20°C to yield a precipitate of unreacted calcium hydroxide and two liquid layers, a thin, upper layer containing mainly methanol, and a main, lower layer containing xylene and overbased calcium salts of the branched C₁₄-C₁₉-alkylmercaptoacetic acids. The upper methanol layer was removed, followed by decantation of the lower xylene layer (2940g). Analysis of the xylene layer by acidification with hydrochloric acid in isopropanol and then titration with 0.5N potassium hydroxide in isopropanol showed it to contain 2.93%w calcium and the basicity index of the overbased calcium salts to be 2.84. 903g "HVI 60" base oil were then added to the xylene layer and the xylene removed from the oil mixture by distillation at 140°C and 100Pa pressure to afford an oil concentrate containing the overbased calcium salts. The oil concentrate was found to have a calcium content of 5.88%w and a viscosity at 100°C of 10.0 mm²/s. The basicity index of the overbased calcium salts was 2.82.

### Example 3

### Stability of Luboil Compositions

In order to assess the formation of haze and/or deposits, 15g samples of the oil concentrates obtained in Example 1(ii) and Example 2(ii) above were each diluted with 85g of a mixture of "HVI 160B" base oil (a bright and clear high viscosity index base oil having kinematic viscosity at 100°C of 10.7 to 11.8 mm²/s (ASTM D445) and minimum flash point of 218 to 228°C (ASTM D92)) and "HVI 650" base oil (a bright and clear high viscosity index base oil having kinematic viscosity at 100°C of 30.5 to 33.5 mm²/s (ASTM D445) and minimum flash point of 267°C (ASTM D92)), the mass ratio of "HVI 160B" base oil to "HVI 650" base oil in the mixture being 3.25:1. The resulting compositions were stored in tubes at 100°C and after 2 and 7 days they were visually assessed for amount of deposits and for brightness. The amount of deposits was expressed in % by volume. The brightness was classified as "bright" (B) or "hazy" (H). The results obtained are shown in Table I below.

**Table I**

| Example No. | Luboil Stability* | |
|---|---|---|
| | 2 Days | 7 Days |
| 1 | 0/B | 0/B |
| 2 | 0/B | 0/B |

| | | |
|---|---|---|
| * expressed are % v/v deposits/brightness | | |

### Example 4

### Engine Test Performance

The oil concentrate obtained in Example 2(ii) above was blended in an amount giving 4.7%w overbased calcium salts in a base oil containing an additive package comprising VI (viscosity index) improver, ashless dispersant, zinc-based anti-wear additive and polymethacrylate pour-point depressant. The resulting oil was evaluated according to sequence 5E ASTM (as described in "Sequence 5E test procedure", 7th draft dated 19th May 1988; ASTM Monitoring Centre, 4400 5th Avenue, Pittsburgh, USA). The sequence 5E test is a gasoline engine test required for the API SG lubricant specification. It tests the ability of an oil to control sludge and varnish deposits and valve train wear in a 2.3 litre Ford four cylinder gasoline engine run under a cyclic operating procedure designed to simulate stop-go running conditions. The results obtained are shown in Table II following:

**Table II**

| Example No. | Engine Test | | |
|---|---|---|---|
| | AES | AEV | ACW inches x 10⁻³ (m x 10⁻⁶) |
| 2 | 9.47 | 8.41 | 5.90 (149.86) |

- AES =: Average Engine Sludge ) (Scale 0 to 10, where
- AEV =: Average Engine Varnish ) 10 represents zero sludge or varnish)
- ACW =: Average Cam Lobe Wear

## Claims

1. An alkaline earth metal salt selected from calcium and magnesium salts of a mercaptocarboxylic acid derivative of the general formula
R-S-(CR¹R²)ₙ-COOH (I)
in which n is 1 to 10; R represents an alkyl group, optionally substituted by a cycloalkyl, heterocyclyl or aryl group; or a cycloalkyl group optionally substituted by an alkyl group; and R¹ and R² are independently selected from hydrogen atoms, and hydroxyl, carboxyl, alkyl, alkoxy, alkylthio, cycloalkyl, aralkyl and aryl groups; subject to the total number of carbon atoms in the derivative being in the range from 12 to 50; with the proviso that the salt excludes calcium salts in which (a) n is 1, R¹ and R² are each a hydrogen atom and R is a C₈, C₁₂ or C₂₀ alkyl group or a benzyl group; (b) n is 2, each R¹ and R² is a hydrogen atom and R is a C₁₂ or C₁₆ alkyl group; and (c) n is 2, each R¹ is a hydrogen atom, the R² on the carbon atom adjacent to the sulphur atom is a hydrogen atom and the other R² is a methyl group, and R is a C₁₂ alkyl group, and excludes magnesium salts in which (a) n is 1, R¹ and R² are each a hydrogen atom and R is a C₁₆ alkyl group; (b) n is 2, each R¹ and R² is a hydrogen atom and R is a C₈, C₁₂ or C₁₈ alkyl group or a benzyl group; and (c) n is 2, each R¹ is a hydrogen atom, the R² on the carbon atom adjacent to the sulphur atom is a hydrogen atom and the other R² is a methyl group, and R is a C₁₂ or C₁₈ alkyl group.

2. A salt as claimed in claim 1, wherein, in formula I, R represents a C₁-C₂₀ alkyl group, optionally substituted by one or more substituents selected from C₃-C₈ cycloalkyl, tetrahydrofuranyl, furanyl, piperidinyl, pyridinyl, tetrahydrothiophenyl, thiophenyl, phenyl and naphthyl groups; or a C₃-C₈ cycloalkyl group optionally substituted by one or more C₁-C₁₀ alkyl groups.

3. A salt as claimed in claim 1 or claim 2, wherein, in formula I, R represents a C₁₀-C₂₀ alkyl group, optionally substituted by one or two substituents selected from C₃-C₆ cycloalkyl, tetrahydrofuranyl, furanyl, piperidinyl, pyridinyl, tetrahydrothiophenyl, thiophenyl, phenyl and naphthyl groups; or a C₃-C₆ cycloalkyl group optionally substituted by one or two C₁-C₆ alkyl groups.

4. A salt as claimed in any one of claims 1 to 3, wherein, in formula I, R¹ and R² are independently selected from hydrogen atoms, and hydroxyl, carboxyl, C₁-C₁₀ alkyl, C₁-C₁₀ alkoxy, C₁-C₁₀ alkylthio, C₃-C₈ cycloalkyl, benzyl, phenyl and naphthyl groups.

5. A salt as claimed in any one of the preceding claims, wherein, in formula I, R¹ and R² are independently selected from hydrogen atoms, and hydroxyl, carboxyl, C₁-C₆ alkyl, C₁-C₆ alkoxy, C₁-C₆ alkylthio, C₃-C₆ cycloalkyl, benzyl, phenyl and naphthyl groups.

6. A salt as claimed in any one of the preceding claims, wherein, in formula I, n is 1, R represents a C₁₄-C₁₉ alkyl group and each of R¹ and R² represents a hydrogen atom.

7. A salt as claimed in any one of the preceding claims which is a calcium salt.

8. A process for the preparation of an alkaline earth metal salt selected from calcium and magnesium salts of a mercaptocarboxylic acid derivative of formula I as claimed in any one of the preceding claims, which comprises reacting a mercaptocarboxylic acid derivative of formula I as defined in any one of the preceding claims with an oxide or hydroxide of calcium or magnesium.

9. A lubricating oil composition comprising a major proportion of a lubricating oil and a minor proportion of an alkaline earth metal salt selected from calcium and magnesium salts of a mercaptocarboxylic acid derivative of formula I as claimed in any one of claims 1 to 7.

10. A lubricating oil concentrate comprising from 10 to 80%w, based on the total concentrate, of an alkaline earth metal salt selected from calcium and magnesium salts of a mercaptocarboxylic acid derivative of formula I as claimed in any one of claims 1 to 7.

## Patentansprüche

1. Erdalkalimetallsalz, ausgewählt aus Calciumund Magnesiumsalzen eines Mercaptocarbonsäurederivats der allgemeinen Formel
R-S-(CR¹R²)ₙ-COOH (I),
worin n für 1 bis 10 steht, R eine gegebenenfalls durch eine Cycloalkyl-, heterocyclische oder Arylgruppe substituierte Alkylgruppe oder eine gegebenenfalls durch eine Alkylgruppe substituierte Cycloalkylgruppe bedeutet und R¹ und R² unabhängig voneinander aus Wasserstoffatomen und Hydroxyl-, Carboxyl-, Alkyl-, Alkoxy-, Alkylthio-, Cycloalkyl-, Aralkyl- und Arylgruppen ausgewählt sind, wobei die Summe der Kohlenstoffatome des Derivats im Bereich von 12 bis 50 liegt, ausgenommen Calciumsalze, in denen (a) n 1 ist, R¹ und R² jeweils ein Wasserstoffatom bedeuten und R eine C₈-, C₁₂- oder C₂₀-Alkylgruppe oder eine Benzylgruppe darstellt; (b) n 2 ist, R¹ und R² jeweils ein Wasserstoffatom bedeuten und R eine C₁₂- oder C₁₆-Alkylgruppe darstellt; und (c) n 2 ist, R¹ jeweils ein Wasserstoffatom bedeutet, R² an dem dem Schwefel benachbarten Kohlenstoffatom ein Wasserstoffatom und das andere R² eine Methylgruppe bedeutet und R eine C₁₂-Alkylgruppe darstellt, und Magnesiumsalze, in denen (a) n 1 ist, R¹ und R² jeweils ein Wasserstoffatom bedeuten und R eine C₁₆-Alkylgruppe darstellt; (b) n 2 ist, R¹ und R² jeweils ein Wasserstoffatom bedeuten und R eine C₈-, C₁₂- oder C₁₈-Alkylgruppe oder eine Benzylgruppe darstellt; und (c) n 2 ist, R¹ jeweils ein Wasserstoffatom bedeutet, R² an dem dem Schwefel benachbarten Kohlenstoffatom ein Wasserstoffatom und das andere R² eine Methylgruppe bedeutet und R eine C₁₂-oder C₁₈-Alkylgruppe darstellt.

2. Salz nach Anspruch 1, wobei in der Formel I R eine gegebenenfalls durch einen oder mehrere Substituenten, ausgewählt aus C₃-C₈-Cycloalkyl-, Tetrahydrofuranyl-, Furanyl-, Piperidinyl-, Pyridinyl-, Tetrahydrothiophenyl-, Thiophenyl-, Phenyl- und Naphthylgruppen, substituierte C₁-C₂₀-Alkylgruppe oder eine gegebenenfalls durch eine oder mehrere C₁-C₁₀-Alkylgruppen substituierte C₃-C₈-Cycloalkylgruppe bedeutet.

3. Salz nach Anspruch 1 oder 2, wobei in der Formel I R eine gegebenenfalls durch einen oder zwei Substituenten, ausgewählt aus C₃-C₆-Cycloalkyl-, Tetrahydrofuranyl-, Furanyl-, Piperidinyl-, Pyridinyl-, Tetrahydrothiophenyl-, Thiophenyl-, Phenyl- und Naphthylgruppen, substituierte C₁₀-C₂₀-Alkylgruppe oder eine gegebenenfalls durch eine oder zwei C₁-C₆-Alkylgruppen substituierte C₃-C₆-Cycloalkylgruppe bedeutet.

4. Salz nach einem der Ansprüche 1 bis 3, wobei in der Formel I R¹ und R² unabhängig voneinander aus Wasserstoffatomen und Hydroxyl-, Carboxyl-, C₁-C₁₀-Alkyl-, C₁-C₁₀-Alkoxy-, C₁-C₁₀-Alkylthio-, C₃-C₈-Cycloalkyl-, Benzyl-, Phenyl- und Naphthylgruppen ausgewählt sind.

5. Salz nach einem der vorhergehenden Ansprüche, wobei in der Formel I R¹ und R² unabhängig voneinander aus Wasserstoffatomen und Hydroxyl-, Carboxyl-, C₁-C₆-Alkyl-, C₁-C₆-Alkoxy-, C₁-C₆-Alkylthio-, C₃-C₆-Cycloalkyl-, Benzyl-, Phenyl- und Naphthylgruppen ausgewählt sind.

6. Salz nach einem der vorhergehenden Ansprüche, I wobei in der Formel I n 1 ist, R eine C₁₄-C₁₉-Alkylgruppe darstellt und R¹ und R² jeweils ein Wasserstoffatom bedeuten.

7. Salz nach einem der vorhergehenden Ansprüche, bei dem es sich um ein Calciumsalz handelt.

8. Verfahren zur Herstellung eines Erdalkalimetallsalzes, ausgewählt aus Calcium- und Magnesiumsalzen eines Mercaptocarbonsäurederivats der Formel I nach einem der vorhergehenden Ansprüche, bei dem man ein Mercaptocarbonsäurederivat der Formel I gemäß einem der vorhergehenden Ansprüche mit einem Calcium- oder Magnesiumoxid oder -hydroxid umsetzt.

9. Schmierölzusammensetzung, enthaltend einen größeren Anteil eines Schmieröls und einen kleineren Anteil eines Erdalkalimetallsalzes, ausgewählt aus Calcium- und Magnesiumsalzen eines Mercaptocarbonsäurederivats der Formel I nach einem der Ansprüche 1 bis 7.

10. Schmierölkonzentrat, enthaltend 10 bis 80 Gew.-%, bezogen auf das Gesamtkonzentrat, eines Erdalkalimetallsalzes, ausgewählt aus Calcium- und Magnesiumsalzen eines Mercaptocarbonsäurederivats der Formel I nach einem der Ansprüche 1 bis 7.

## Revendications

1. Sel de métaux alcalino-terreux choisi parmi les sels de calcium et de magnésium d'un dérivé d'acide mercaptocarboxylique de la formule générale
R-S-(CR¹R²)ₙ-COOH (I)
dans laquelle n a une valeur qui varie de 1 à 10, R représente un radical alkyle, éventuellement substitué par un groupe cycloalkyle, hétérocyclyle ou aryle; ou un groupe cycloalkyle éventuellement substitué par un radical alkyle; et R¹ et R² représentent indépendamment chacun un atome d'hydrogène, un radical hydroxyle, carboxyle, alkyle, alcoxy, alkylthio, cycloalkyle, aralkyle et aryle; pour autant que le nombre total d'atomes de carbone dans le dérivé fluctue de 12 à 50; avec la condition que soient exclus du sel, les sels de calcium dans lesquels (a) n est égal à 1, R¹ et R² représentent chacun un atome d'hydrogène et R est un groupe alkyle en C₈, C₁₂ ou C₂₀ ou un groupe benzyle; (b) n est égal à 2, chaque symbole R¹ et R² représentent un atome d'hydrogène er R représente un groupe alkyle en C₁₂ ou C₁₆, le symbole R² sur l'atome de carbone voisin de l'atome de soufre est un atome d'hydrogène et l'autre symbole R² est un groupe méthyle et R est un groupe alkyle en C₁₂ et que soient exclus du sel, les sels de magnésium dans lesquels (a) n est égal à 1, R¹ et R² représentent chacun un atome d'hydrogène et R est un groupe alkyle; (b) n est égal à 2, chaque symbole R¹ et R² représentent un atome d'hydrogène et R est un groupe alkyle en C₈, C₁₂ ou C₁₈ ou un groupe benzyle; et (c) n est égal à 2, chaque symbole R¹ représente un atome d'hydrogène, le symbole R² sur l'atome de carbone voisin de l'atome de soufre est un atome d'hydrogène et l'autre symbole R² est un groupe méthyle et R est un groupe alkyle en C₁₂ ou C₁₈.

2. Sel suivant la revendication 1, caractérisé en ce que dans la formule I, R représente un groupe alkyle en C₁ à C₂₀, éventuellement substitué par un ou plusieurs substituants choisis parmi les radicaux cycloalkyle en C₃ à C₈, tétrahydrofurannyle, furannyle, pipéridinyle, pyridinyle, tétrahydrothiophényle, thiophényle, phényle et naphtyle; ou un radical cycloalkyle en C₃ à C₈, éventuellement substitué par un ou plusieurs groupes alkyle en C₁ à C₁₀.

3. Sel suivant la revendication 1 ou la revendication 2, caractérisé en ce que dans la formule I, R représente un groupe alkyle en C₁₀ à C₂₀, éventuellement substitué par un ou deux substituants choisis parmi les radicaux cycloalkyle en C₃ à C₆, tétrahydrofurannyle, furannyle, pipéridinyle, pyridinyle, tétrahydrothiophényle, thiophényle, phényle et naphtyle; ou un groupe cycloalkyle, éventuellement substitué par un ou deux radicaux alkyle en C₁ à C₆.

4. Sel suivant l'une quelconque des revendications 1 à 3, caractérisé en ce que dans la formule I, R¹ et R² sont indépendamment choisis parmi les atomes d'hydrogène et les radicaux hydroxyle, carboxyle, alkyle en C₁ à C₁₀, alcoxy en C₁ à C₁₀, alkylthio en C₁ à C₁₀, cycloalkyle en C₃ à C₈, benzyle, phényle et naphtyle.

5. Sel suivant l'une quelconque des revendications précédentes, caractérisé en ce que dans la formule I, R¹ et R² sont indépendamment choisis parmi les atomes d'hydrogène et les radicaux hydroxyle, carboxyle, alkyle en C₁ à C₆, alcoxy en C₁ à C₆, alkylthio en C₁ à C₆, cycloalkyle en C₃ à C₆, benzyle, phényle et naphtyle.

6. Sel suivant l'une quelconque des revendications précédentes, caractérisé en ce que dans la formule I, n est égal à 1, R représente un groupe alkyle en C₁₄ à C₁₉ et chacun des symboles R¹ et R² représente un atome d'hydrogène.

7. Sel suivant l'une quelconque des revendications précédentes, caractérisé en ce qu'il est un sel de calcium.

8. Procédé de préparation d'un sel de métal alcalino-terreux choisi parmi les sels de calcium et de magnésium d'un dérivé d'acide mercaptocarboxylique de la formule I suivant l'une quelconque des revendications précédentes, caractérisé en ce que l'on fait réagir un dérivé d'acide mercaptocarboxylique de la formule I tel que défini dans l'une quelconque des revendications précédentes, avec un oxyde ou hydroxyde de calcium ou de magnésium.

9. Composition d'huile lubrifiante comprenant une proportion majeure d'une huile lubrifiante et une proportion mineure d'un sel de métal alcalino-terreux choisi parmi les sels de calcium et de magnésium d'un dérivé d'acide mercaptocarboxylique de la formule I tel que défini dans l'une quelconque des revendications 1 à 7.

10. Concentré d'huile lubrifiante comprenant de 10 à 80% en poids, sur base du concentré total, d'un sel de métal alcalino-terreux choisi parmi les sels de calcium et de magnésium d'un dérivé d'acide mercaptocarboxylique de la formule I suivant l'une quelconque des revendications 1 à 7.
